(19)

**Europäisches Patentamt**

**European Patent Office**

**Office européen des brevets**

(11) **EP 4 732 831 A1**

(12) **EUROPEAN PATENT APPLICATION**

(43) Date of publication:
29.04.2026 Bulletin 2026/18

(21) Application number: 24208829.2

(22) Date of filing: 25.10.2024

(51) International Patent Classification (IPC):
**A61K 9/10** (2006.01)        **A61K 47/18** (2017.01)
**A61K 47/32** (2006.01)

(52) Cooperative Patent Classification (CPC):
**A61K 47/183; A61K 9/0053; A61K 9/146;
A61K 47/32**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Merck Patent GmbH
64293 Darmstadt (DE)**

(72) Inventor: **The designation of the inventor has not
yet been filed**

(74) Representative: **Merck Patent Association
Merck Patent GmbH
64271 Darmstadt (DE)**

(54) **TERNARY SYSTEMS WITH IMPROVED DRUG LOADING CAPACITY, PHYSICAL STABILITY AND PRECIPITATION INHIBITION**

(57) The invention relates to a pharmaceutical composition comprising an active pharmaceutical ingredient (API), a carrier polymer, and tryptophan; as well as to a pharmaceutical composition according to the invention for use as a medicament. The invention further relates to a method for producing a pharmaceutical composition comprising the steps of a) mixing an API and a carrier polymer, and b) adding tryptophan.

EP 4 732 831 A1

## Description

### Field of the invention

The invention relates to pharmaceutical compositions with improved physical properties comprising tryptophan.

### Technical background

The majority of newly discovered drugs are poorly soluble and belong to the Biopharmaceutic Classification System (BCS) class II. Low solubility is a significant disadvantage for a drug since this also means low oral absorption and bioavailability.

A common strategy to enhance solubility and bioavailability of these compounds is the formulation of amorphous solid dispersions (ASD). ASDs are mixtures of an active pharmaceutical ingredient (API) and a carrier polymer in which the API is in an amorphous or non-crystalline state.

Unlike crystal forms of a drug, which are highly ordered and highly stable, ASDs are metastable. The high-energy amorphous state typically exhibits improved solubility and bioavailability of the API due to the absence of a crystal lattice that needs to be broken down in order for the drug to dissolve in water.

An ASD can be obtained by a variety of techniques among which hot melt extrusion (HME) is widely used. During HME, an API-polymer mixture is blended in a heated barrel and the API can become amorphous by either heating above the melting temperature and/or dissolving in the polymer matrix. Other than HME, spray-drying and ball-milling are commonly applied techniques for the production of ASDs.

Recently, the use of co-formers has been described in scientific literature. Particularly, the use of amino acids as co-formers has been described for ball milling processes.

ASDs formulations usually consist of a carrier polymer and the API. The solubility in the polymer is often limited, which is why high drug loads are difficult to achieve. Drug loading capacity is the amount of API that can be contained in the formulation per mass of the carrier polymer. It may also be understood as the ratio of mass of API contained in the carrier polymer phase in relation to the carrier polymer mass. In the case of APIs that require a high dose, high drug loaded ASDs are crucial to achieve an adequate tablet size which is still swallowable. Therefore, it is important to find methods that allow a higher drug loading capacity of the ASD.

As the amorphous state is inherently unstable, the drug will eventually recrystallize, in turn resulting in poor solubility and bioavailability. Especially at high drug loadings, the API is more likely to recrystallize. Therefore, it is important to find ways to enhance physical stability of pharmaceutical compositions and prevent recrystallization, especially for high drug loaded ASDs.

Drug supersaturation in biorelevant media is essential to achieve optimal drug efficacy and bioavailability. Supersaturation occurs when the dissolved concentration of the drug in the biorelevant medium exceeds the equilibrium solubility of the crystal form. However, maintaining drug supersaturation from ASDs is a challenge as the amorphous form of the API in ASDs is at a higher energy state than its crystalline form, meaning that it has a strong tendency to revert back to its lower energy crystal form. This process is known as precipitation or crystallization, which can result in a reduction in drug solubility, bioavailability, and efficacy. Additionally, drug loading can significantly affect precipitation in supersaturated solutions. In general, higher drug loading in ASDs leads to a higher likelihood of precipitation due to the increased saturation of the dissolution medium. Therefore, it is also important to find methods that allow a high drug loading and maintenance of the supersaturated state at the same time.

### Objective problem to be solved

To address these challenges, pharmaceutical compositions are required which provide a high drug loading capacity and enhanced physical stability of an ASD, while at the same time preventing recrystallization and maintaining drug supersaturation in a biorelevant medium.

### Summary of the invention

In one aspect, the invention relates to a pharmaceutical composition comprising an active pharmaceutical ingredient (API), a carrier polymer, and tryptophan.

In a second aspect, the invention relates to a pharmaceutical composition according to the invention for use as a medicament.

In a third aspect, the invention relates to a method for producing a pharmaceutical composition comprising the steps of a) mixing an API and a carrier polymer, and b) adding tryptophan.

**Brief description of the figures**

**Fig. 1** shows the DSC thermograms of a binary sample containing carvedilol and PVA 4-88 processed via vacuum compression molding after preparation and after storage for one week at 25 °C and 60% r.h. and 40 °C and 75% r.h. at open and closed conditions.

**Fig. 2** shows the DSC thermograms of a ternary sample containing carvedilol, tryptophan and PVA 4-88 processed via vacuum compression molding after preparation and after storage for one week at 25 °C and 60% r.h. and 40 °C and 75% r.h. at open and closed conditions.

**Fig. 3** compares the dissolution profiles in FaSSIF at pH 6.5 and 37 °C for the binary sample with PVA 4-88 and carvedilol and the corresponding ternary sample with tryptophan.

**Fig. 4** compares the dissolution profiles in FaSSIF at pH 6.5 and 37 °C for the binary sample with PVA 4-88 and ketoconazole, the corresponding ternary sample with tryptophan, and of crystalline ketoconazole.

**Fig. 5** compares the dissolution profiles in FaSSIF at pH 6.5 and 37 °C for the ternary sample with PVA 4-88, ketoconazole and tryptophan being molten together compared to a ternary mixture with identical composition in which the tryptophan was added after melting of the sample.

**Fig. 6** compares the dissolution profiles in FaSSIF at pH 6.5 and 37 °C for the binary sample with copovidone and ketoconazole, the corresponding ternary sample with tryptophan, and of crystalline ketoconazole.

**Fig. 7** compares the dissolution profiles in FaSSIF at pH 6.5 and 37 °C for the binary sample with PVA 3-82 and ketoconazole, the corresponding ternary sample with tryptophan, and of crystalline ketoconazole.

**Fig. 8** compares the DSC thermograms of naproxen in a binary and ternary sample processed via vacuum compression molding and the physical mixture of the binary sample.

**Fig. 9** compares the dissolution profiles in $SGF_{sp}$ and 37 °C for the binary sample with PVA 4-88 and naproxen, the corresponding ternary sample with tryptophan, and crystalline naproxen.

**Fig. 10** compares the dissolution profiles in $SGF_{sp}$ and 37 °C for the binary sample with copovidone and naproxen, the corresponding ternary sample with tryptophan, and crystalline naproxen.

**Fig. 11** compares the dissolution profiles in $SGF_{sp}$ and 37 °C for the binary sample with copovidone and naproxen, the corresponding ternary sample with tryptophan or proline respectively, and crystalline naproxen.

**Fig. 12** compares the dissolution profiles in $SGF_{sp}$ and 37 °C for the binary sample with PVA 4-88 and felodipine, the corresponding ternary sample with tryptophan, and crystalline felodipine.

**Fig. 13** compares the dissolution profiles in FaSSIF at pH 6.5 and 37 °C for the binary sample with PVA 4-88 and carvedilol and the corresponding ternary sample with proline.

**Detailed description of the invention**

In one aspect, the invention relates to a pharmaceutical composition comprising an active pharmaceutical ingredient (API), a carrier polymer, and tryptophan. Compared to a binary mixture of API and carrier polymer, a ternary mixture of API, carrier polymer and tryptophan has improved drug loading capacity and precipitation inhibition, since the presence of the amino acid tryptophan as a co-former in the pharmaceutical composition has advantageous effects on the physical stability of the amorphous form, inhibiting precipitation of the API into its crystal form. This in turn allows for higher drug efficacy and bioavailability, even at high-dose drugs.

In one embodiment, the API and the carrier polymer are present at a w/w% ratio of about 1:99 to about 50:50, and tryptophan is present at a w/w% concentration of about 1% to about 30% of the total composition, preferably of about 10% to about 30% of the total composition, more preferably of about 20% to about 30% of the total composition, most preferably at an equimolar concentration with respect to the API. As demonstrated in the examples below, the pharmaceutical composition shows improved drug loading capacity, physical stability and precipitation inhibition at an ample range of tryptophan concentrations, but increasingly advantageous results are achieved when the tryptophan concentration approaches about 30% w/w of the total composition, and optimal results are achieved when tryptophan is at an equimolar concentration with respect to the API.

As used herein, the term "about" is used synonymously with the term "approximately". In some embodiments, the term "about" includes the indicated value plus or minus up to 10% of said value. Such values are encompassed by the scope of the claims when including the term "about".

In some embodiments, the composition is capable of maintaining supersaturation of the API in a biorelevant medium.

As used herein, the term "supersaturation" refers to a metastable state of an aqueous solution or suspension with a concentration of a solute that exceeds the equilibrium solubility of the solute.

As used herein, the term "biorelevant medium" refers to an aqueous solution or suspension of composition and physicochemical characteristics that resemble fluids found along the different portions of the human gastrointestinal tract.

The capacity to maintain drug supersaturation in a biorelevant medium is crucial to achieve optimal gastrointestinal absorption of drugs with low solubility, which is in turn important for drug efficacy and bioavailability.

As used herein, a drug having "low solubility" is difficult to dissolve in aqueous solutions such as gastrointestinal fluids, which reduces the total amount of the drug that can be effectively absorbed into the bloodstream from solid oral forms, thus limiting bioavailability. According to the Biopharmaceutics Classification System (BCS), a drug is classified as highly soluble if the highest single therapeutic dose is completely soluble in 250 mL or less of aqueous media over a pH range of 1.2 to 6.8 at $37 \pm 1°C$. If a drug does not satisfy this criterion at the highest single therapeutic dose, then it is considered as having low solubility. BCS is a classification system categorizing drugs based on solubility and permeability properties.

In a preferred embodiment, the pharmaceutical composition is formulated as an amorphous solid dispersion (ASD).

As used herein, the term "amorphous solid dispersion (ASD)" refers to a mixture of two or more components in a solid state, wherein an amorphous API is dispersed in an amorphous carrier polymer. In an ideal ASD, a single homogeneous phase is formed, wherein the API is mixed at a molecular level within the carrier polymer, forming strong interactions between API and carrier polymer. Parameters according to which solid dispersions are classified are 1) The physical states and molecular arrangement of the API, i.e. API dispersed in a crystalline state (in the form of crystalline particles), API dispersed in an amorphous state (in the form of amorphous clusters) or API molecularly dispersed within the carrier, and 2) The physical states and molecular arrangement of the carrier, i.e. crystalline or amorphous. The physical state of both the API and carrier and their molecular arrangement significantly influence the stability as well as dissolution behavior of the system. ASDs are mixtures of API and carrier in which the API is in an amorphous or non-crystalline state. According to parameters 1) and 2), ASDs can be Class A-C (amorphous API dispersed in crystalline carrier), Class A-A (amorphous API dispersed in amorphous carrier), Class M-C (API molecularly dispersed in crystalline carrier), or Class M-A (API molecularly dispersed in amorphous carrier).

When the pharmaceutical composition according to the invention is formulated as an ASD, the API is in an amorphous or non-crystalline state, as opposed to a crystal form which is highly ordered. The lack of long-range order and a crystal lattice that needs to be broken down in order for the drug to dissolve in water makes compositions formulated as ASDs more soluble compared to crystal forms. The amorphous form of an API has a higher, metastable energy state compared to that of the crystalline form.

In yet another embodiment, the pharmaceutical composition is capable of preventing recrystallization of the API in the amorphous solid dispersion (ASD). In a typical binary ASD, the interactions between the API and the carrier polymer ideally inhibit crystal nucleation or growth of the amorphous API phase. However, since the amorphous state is inherently unstable, the API will eventually recrystallize and amorphous-amorphous phase separation will occur, reducing solubility and therefore bioavailability. Crucially, the API is more likely to recrystallize in a binary ASD at high drug loadings due to the increased saturation of the dissolution medium, which causes problems especially for high-dose drugs. The composition according to the invention prevents precipitation of the API back to its crystal form, thereby enhancing the drug loading capacity and allowing even high-dose drugs to maintain supersaturation in a biorelevant medium.

In some embodiments, the API is a Biopharmaceutics Classification System (BCS) Class II drug, a BCS Class IV drug, or any drug having low solubility. Specifically, BCS class II drugs are characterized by high gastrointestinal permeability but low solubility, whereas BCS class IV drugs are characterized by both low gastrointestinal permeability and low solubility.

In a preferred embodiment, the carrier polymer is a polyvinyl alcohol (PVA). As demonstrated in the examples below, PVA as the carrier polymer is particularly advantageous for the physical stability and maintenance of supersaturation of the composition according to the invention.

PVA is a synthetic water-soluble polymer that has the idealized formula $[CH_2CH(OH)]_n$. It possesses good film-forming, adhesive, and emulsifying properties. PVA is prepared from polyvinyl acetate, where the functional acetate groups are either partially or completely hydrolysed to alcohol functional groups. If not completely hydrolysed, PVA is a random copolymer consisting of vinyl alcohol repeat units -[CH2CH(OH)]- and vinyl acetate repeat units -[CH2CH(OOCCH3)]-. The polarity of PVA is closely linked to its molecular structure. The hydrolysis degree and the molecular weight determine the molecular properties of PVA. As the degree of hydrolysis of acetate groups increases, the solubility of the polymer in aqueous media and also crystallinity and melting temperature of the polymer increase. However, at high hydrolysis degrees over 88%, the solubility of PVA decreases again. PVA is generally soluble in water, but almost insoluble in almost all organic solvents, excluding, in some cases, ethanol.

The typical PVA nomenclature indicates the viscosity of a 4% solution at 20°C and the degree of hydrolysis of the polymer. For example, PVA 4-88 is a PVA grade with a viscosity of 4 mPas that is 88% hydrolysed, i.e. having 88% of vinyl alcohol repeat units and 12% of vinyl acetate repeat units. A skilled person is aware that a hydrolysis grade of 88% and a viscosity of 4 mPas encompasses calculated hydrolysis grades of 87,50% to 88,49% and calculated viscosities of 3,50 mPas to 4,49 mPas% according to common rounding methods. Viscosity is measured as stated in US Pharmacopeia 39 under Monograph "Polyvinyl Alcohol" with the method Viscosity-Rotational Method (912). The degree of hydrolysis is measured by determining the saponification value of the PVA, e.g. as stated in US Pharmacopeia 39 under Monograph "Polyvinyl Alcohol" under "Degree of Hydrolysis":

Sample: 1 g of PVA, previously dried at 110° to constant weight

Analysis: Transfer the Sample to a wide-mouth, 250-ml conical flask fitted by means of a suitable glass joint to a reflux condenser. Add 35 ml of dilute methanol (3 in 5), and mix gently to ensure complete wetting of the solid. Add 3 drops of phenolphthalein TS, and add 0.2 N hydrochloric acid or 0.2 N sodium hydroxide if necessary, to neutralize. Add 25.0 ml of 0.2 N sodium hydroxide VS, and reflux gently on a hot plate for 1 h. Wash the condenser with 10 ml of water, collecting the washings in the flask, cool, and titrate with 0.2 N hydrochloric acid VS. Concomitantly perform a blank determination in the same manner, using the same quantity of 0.2 N sodium hydroxide VS.

Calculate the saponification value:

$$Result = [(VB - VS) \times N \times Mr]/W$$

VB = volume of 0.2 N hydrochloric acid VS consumed in the titration of the blank (ml)

VS = volume of 0.2 N hydrochloric acid VS consumed in the titration of the Sample solution (ml)

N = actual normality of hydrochloric acid VS

Mr = molecular weight of potassium hydroxide, 56.11

W = weight of the portion of PVA taken (g)

Calculation of degree of hydrolysis:
Calculate the degree of hydrolysis, expressed as a percentage of hydrolysis of polyvinyl acetate:

$$Result = 100 - [7.84 \times S/(100 - 0.075 \times S))$$

S = saponification value of the PVA

The use of PVA grades is of interest for the formulation of solid oral pharmaceutical dosage forms with an instant, immediate or prolonged API release.

In some embodiments, the PVA has a viscosity of a 4% solution at 20°C of 3 mPas to 8 mPas, more preferably a viscosity of a 4% solution at 20°C of 3 mPas to 5 mPas, most preferably a viscosity of a 4% solution at 20°C of 3 mPas to 4 mPas.

In other embodiments, the PVA has a hydrolysis degree of 70% to 90%, preferably 80% to 90% and a viscosity as mentioned above.

In some embodiments, the PVA has a hydrolysis degree of 70% to 90%, and viscosity of a 4% solution at 20°C of 3 mPas to 8 mPas, more preferably a viscosity of a 4% solution at 20°C of 3 mPas to 5 mPas, most preferably a viscosity of a 4% solution at 20°C of 3 mPas to 4 mPas.

In some embodiments, the PVA has a hydrolysis degree of 80% to 90% and a viscosity of a 4% solution at 20°C of 3 mPas or a hydrolysis degree of 80% to 90% and a viscosity of a 4% solution at 20°C of 4 mPas.

In another embodiment, the PVA is preferably PVA 3-80, PVA 3-82, PVA 4-88, PVA 5-88, PVA 8-88, or PVA 5-74, more preferably PVA 3-80, PVA 3-82 or PVA 4-88, most preferred PVA 4-88.

In a preferred embodiment, the pharmaceutical composition is obtained by hot-melt extrusion (HME) or spray drying (SD). Fusion-based methods such as HME and evaporation-based methods such as SD are routinely used to blend the API and carrier polymer. In HME, the pharmaceutical compositions blended in a heated barrel, whereby the API is made amorphous by heating above the melting temperature and/or dissolving in the polymer matrix. In SD, the pharmaceutical composition is atomized or sprayed into drops of controlled size, from which dry powder is obtained by rapidly drying with a hot gas, thereby obtaining an ASD.

In another aspect of the invention, the pharmaceutical composition according to the invention is for use as a medicament.

In yet another aspect of the invention, the invention relates to a method for producing a pharmaceutical composition, comprising the steps of a) mixing an API and a carrier polymer, and b) adding tryptophan.

In one embodiment, the API and the carrier polymer are mixed in step a) at a w/w% ratio of about 1:99 to about 50:50, and tryptophan is added in step b) at a w/w% concentration of about 1% to about 30% of the total composition, preferably at an equimolar concentration with respect to the API.

In another embodiment, the mixture obtained in step a) is an amorphous solid dispersion (ASD).

In one embodiment, the method is based on hot-melt extrusion (HME) or spray drying (SD).

In another embodiment, the API of step a) in the method is a Biopharmaceutics Classification System (BCS) Class II drug, or a BCS Class IV drug, or any drug presenting low solubility.

In yet another embodiment, the carrier polymer in the method is selected from the group comprising polyvinyl alcohol (PVA).

In another embodiment, the PVA is preferably PVA 3-80, PVA 3-82, PVA 4-88, PVA 5-88, PVA 8-88, or PVA 5-74, more preferably PVA 3-80, PVA 3-82 or PVA 4-88, most preferred PVA 4-88..

In one embodiment, the method further comprises the steps of melting the mixture at about 210 °C under vacuum, cooling the mixture down to room temperature, and milling or cryomilling the resulting melt.

## Examples

### Method 1 (ternary mixtures):

Preparing a physical mixture containing the drug and polymer at a drug:polymer ratio of 25:75 (Example 2) or 40:60 (Example 4, 7, 9, 11, 13, 14, 16) and adding tryptophan at an equimolar concentration (with respect to drug). Melting the physical mixture at an elevated temperature for a defined period of time under vacuum and cooling down to room temperature. Parameters:

| Example | Temperature (°C) | Time (min) |
|---|---|---|
| 2 | 210 | 5 |
| 4, 13, 14 | 210 | 6 |
| 9, 11 | 190 | 6 |
| 7, 16 | 200 | 6 |

Milling:

    a. Milling the cooled melt via ball milling for 0.5 min at 25 Hz (Example 2).
    b. Milling the cooled melt at mild conditions using an IKA Tube Mill for 20 seconds in total at 25,000 rpm (Example 4, 7, 9, 11, 13, 14, 16).

### Method 2 (binary mixtures):

Preparing a physical mixture containing the drug and polymer at a drug:polymer ratio of 25:75 (Example 1) or a ratio corresponding the drug load of the ternary sample (Example 3, 6, 8, 10, 12, 15). Melting the physical mixture at an elevated temperature for a defined period of time under vacuum and cooling down to room temperature. Parameters:

| Example | Temperature (°C) | Time (min) |
|---------|------------------|------------|
| 1 | 230 | 2 |
| 3, 12, | 210 | 6 |
| 8, 10 | 190 | 6 |
| 6,15 | 200 | 6 |

Milling:

a. Milling the cooled melt via ball milling for 0.5 min at 25 Hz (Example 1).
b. Milling the cooled melt using an IKA Tube Mill for 20 seconds in total at 25,000 rpm (Example 3, 6, 8, 10, 12, 15).

**Method 3:**

Preparing a physical mixture containing the drug and polymer at a drug:polymer ratio of 40:60.
Melting the physical mixture at an elevated temperature for a defined period of time under vacuum and cooling down to room temperature.

| Example | Temperature (°C) | Time (min) |
|---------|------------------|------------|
| 5 | 210 | 6 |

Milling the cooled melt using an IKA Tube Mill for 20 seconds in total at 25,000 rpm. Adding tryptophan to the milled powder at an equimolar concentration (with respect to drug).

**Storage stability:**

Storing the samples at 25 °C and 60% r.H. (open and closed) and 40°C/75% r.H. (open and closed) for one week (Example 1 and 2).

**Differential scanning calorimetry**

Analysis of the milled samples utilizing differential scanning calorimetry. Approximately 5 - 10 mg of milled sample was weighted into aluminum pans and sealed hermetically. Prior to analysis pans were pierced. Samples were heated at rate of 10 K/min. Samples were heated from 0 °C - 210 °C (Example 6 and 7) or 0 °C - 220 °C (Example 1) or 20 °C - 220 °C (Example 2).

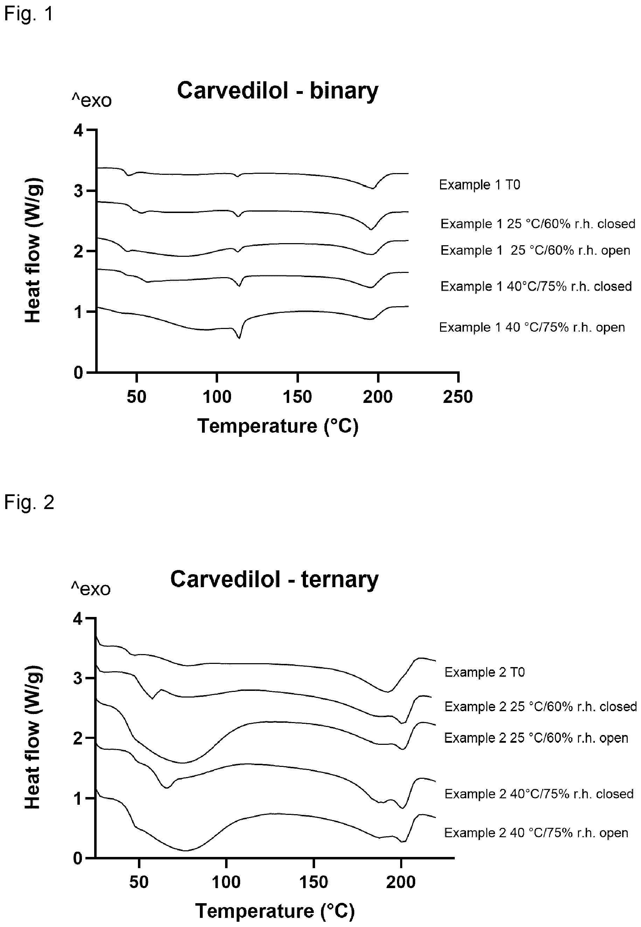

As can be seen in Fig. 1 the binary sample (example 1) shows remaining crystalline API, indicated by the endothermic event at 115 °C, reflecting the melting point of carvedilol. During storage the API recrystallizes, which is indicated by the increase of the peak area reflecting an increase in melting enthalpy.

Fig. 2 shows the DSC thermograms of the ternary sample (example 2) indicating that the API is amorphous and remained amorphous during storage due to the absence of a melting peak in the freshly prepared and stored samples.

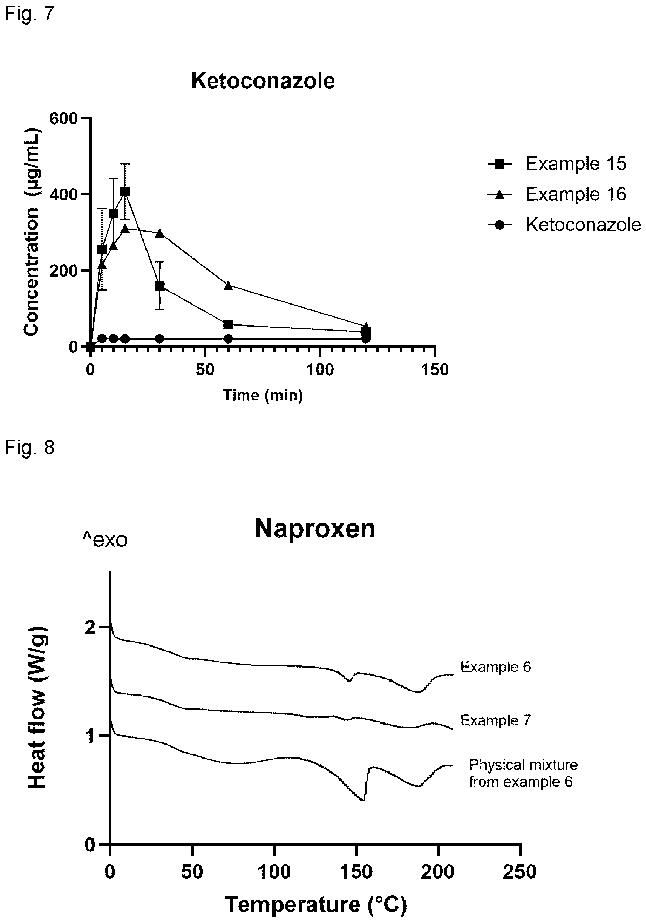

As can be seen in Fig. 8, showing the DSC thermograms of binary, ternary samples containing the API naproxen the binary sample shows remaining crystallinity of the API naproxen, which is indicated by the endothermic event at 150 °C, reflecting the melting point of naproxen. The proportion of crystalline naproxen is reduced in the ternary sample, which is indicated by the decrease of the peak area.

**Non-sink dissolution**

*Example 1 and 2:*

Performing dissolution at non-sink conditions at 37°C in fasted state simulated intestinal fluid (FaSSIF) at pH 6.5. Milled samples were weighed in a range of 100 - 180 mg. FaSSIF volume was adjusted to the respective weight targeting a maximum concentration of 570 $\mu$g/mL. Samples were agitated during analysis at 250 rpm. 2.5 mL of sample was withdrawn at the respective timepoint and filtrated through a 0.2 $\mu$m cellulose acetate filter and diluted with methanol in a 1:1 ratio prior to HPLC analysis.

*Examples 6, 7, 8, 9, 14:*

Performing dissolution at non-sink conditions at 37°C in simulated gastric fluid (SGF$_{sp}$).

Samples were weighed corresponding a drug content of 6 mg. FaSSIF volume was 8 mL.

Samples were agitated during analysis at 450 rpm. 1 mL of sample was withdrawn at the respective timepoint and filtrated through a 0.45 $\mu$m PTFE filter and diluted with a mixture of acetonitrile, purified water and glacial acetic acid (ratio 4.5 : 5.4 : 0.1 ) prior to HPLC analysis.

*Examples 3, 4, 5, 10, 11, 12, 13, 15, 16:*

Performing dissolution at non-sink conditions at 37°C in FaSSIF at pH 6.5. Samples were weighed corresponding a drug content of 6 mg. FaSSIF volume was 8 mL. Samples were agitated during analysis at 450 rpm. 1 mL of sample was withdrawn at the respective timepoint and filtrated through a 0.45 $\mu$m PTFE filter and diluted with a mixture of acetonitrile, purified water and glacial acetic acid (ratio 4.5 : 5.4 : 0.1 ) prior to HPLC analysis.

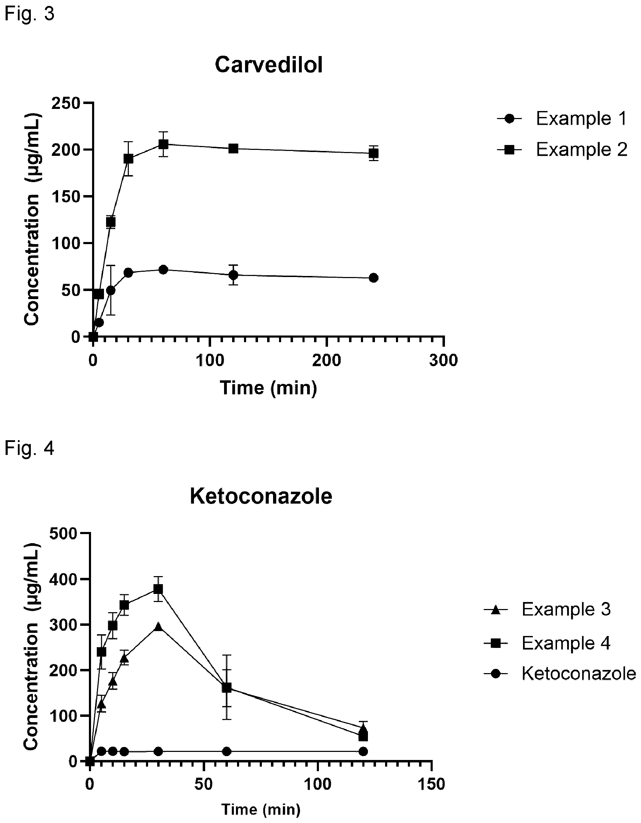

As shown in Fig. 3, 4, 7, 9 and 12 the ternary sample containing PVA and tryptophan reaches a higher supersaturation of the API compared to the binary sample.

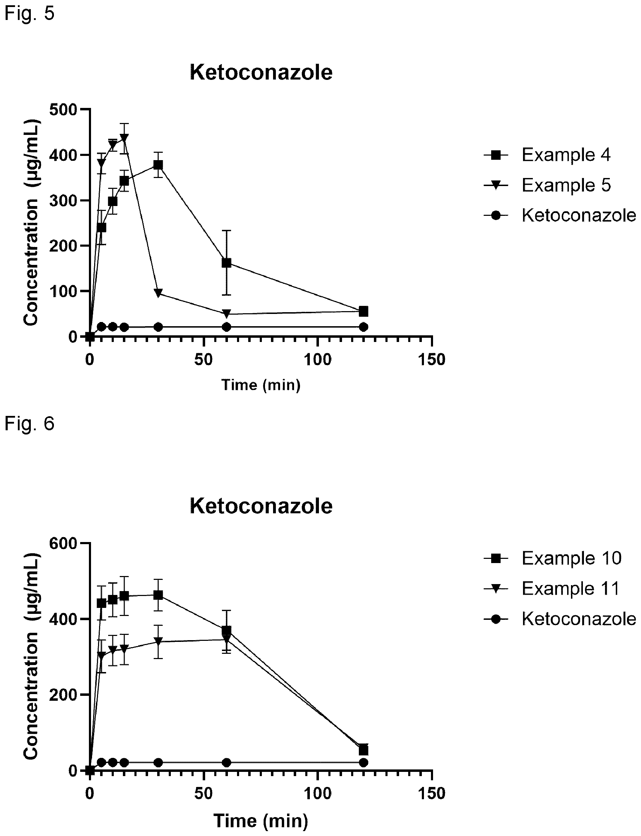

As shown in Fig. 5, the ternary sample in which all three components are molten together reaches a higher supersaturation compared to a sample in which the tryptophan was added by physical mixing to the molten sample.

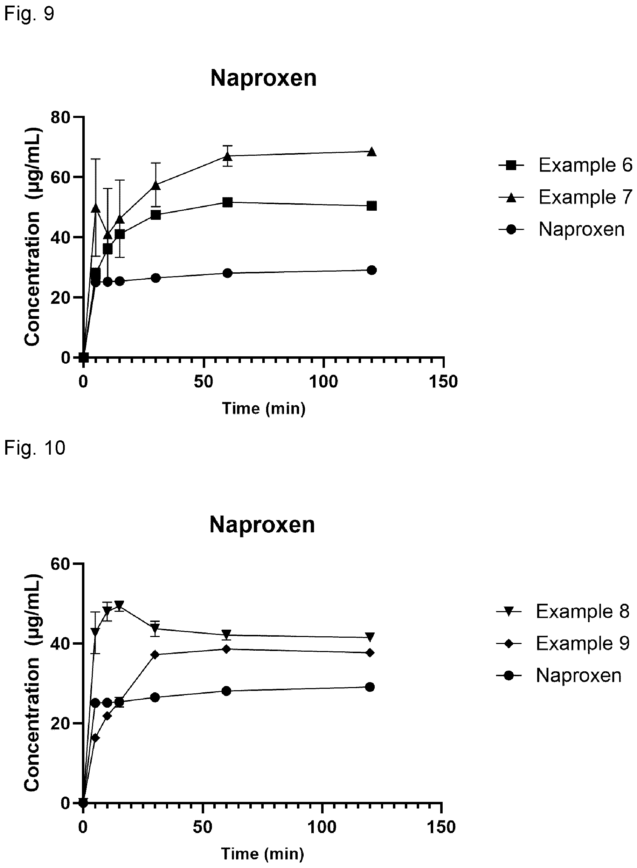

As shown in Fig. 6 and 10 the ternary sample containing copovidone and tryptophan reaches lower concentrations of dissolved API compared to the binary sample.

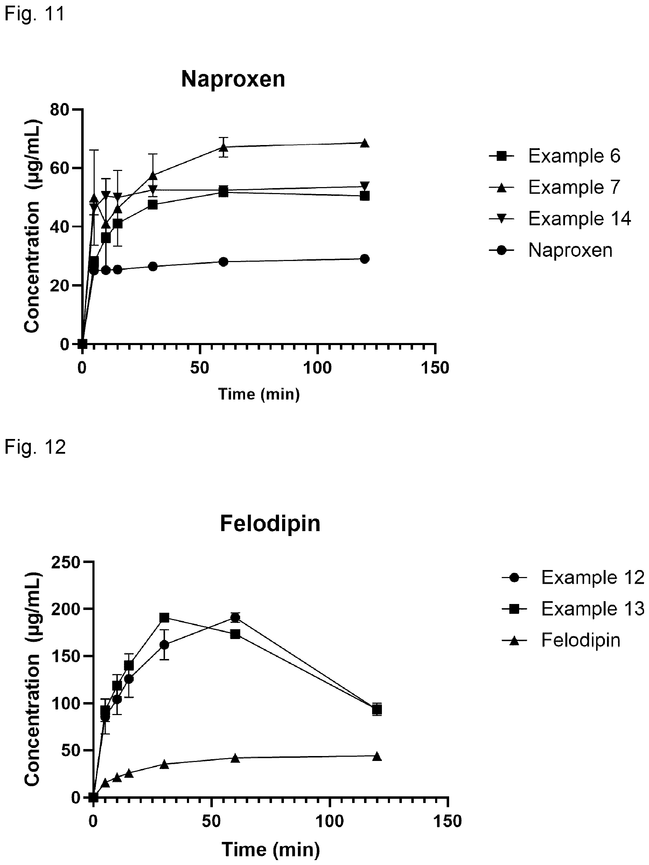

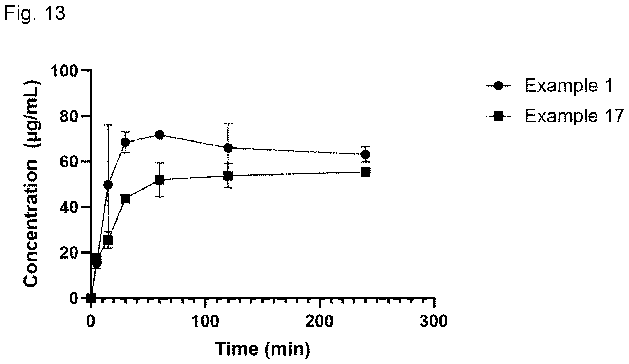

As shown in Fig. 11 and 13 the ternary sample containing proline instead of tryptophan reaches lower concentrations of dissolved API compared to ternary or binary samples.

Preparation of dissolution media:

To prepare the SGF$_{sp}$, first, 800 mL of 1 M HCl was placed in a 10 L flask. Then 20 g of NaCl was weighed into the 10 L flask, dissolved and subsequently filled up to 10 L with deionized water.

To prepare FaSSIF a buffer solution war prepared. For the buffer solution, 4.2 g of sodium hydroxide pellets, 34,38 g of sodium phosphate monobasic anhydrous and 61,86 g of sodium chloride were weighed in a 10 L volumetric flask and filled up with approximately 9 L of purified water and stirred until everything is dissolved. After that, the pH of buffer was checked and adjusted to pH 6.5 with Hydrochloric acid 1 M (adjusted pH = 6.512) and made up to volume (10 L) with purified water. After that 224 mg of FaSSIF powder were weighed and added into a 100 ml volumetric flask. Subsequently 100 ml of the prepared buffer were added to the FaSSiF powder and stirred until everything is dissolved. After 2 h of equilibration time, the FaSSiF solution was ready to use.

*Table 1: Summary of compositions. Ratios in w/w%.*

| Example | PVA 4-88 | PVA 3-82 | Copovidone | Carvedilol | Ketoconazole | Naproxen | Felodipine | Tryptophan | Proline |
|---|---|---|---|---|---|---|---|---|---|
| 1 | 75 | - | - | 25 | - | - | - | - | - |
| 2 | 67 | - | - | 22 | - | - | - | 11 | - |
| 3 | 65 | - | - | - | 35 | - | - | - | - |
| 4 | 52 | - | - | - | 35 | - | - | 13 | - |
| 5 | 52 | - | - | - | 35 | - | - | 13 | - |
| 6 | 70 | - | - | - | - | 30 | - | - | - |
| 7 | 44 | - | - | - | - | 30 | - | 26 | - |
| 8 | - | - | 70 | - | - | 30 | - | - | - |
| 9 | - | - | 44 | - | - | 30 | - | 26 | - |
| 10 | - | - | 65 | - | 35 | - | - | - | - |
| 11 | - | - | 52 | - | 35 | - | - | 13 | - |
| 12 | 70 | - | - | - | - | - | 30 | - | - |
| 13 | 44 | - | - | - | - | - | 30 | 26 | - |
| 14 | 50 | - | - | - | - | 33 | - | - | 17 |
| 15 | - | 65 | - | - | 35 | - | - | - | - |
| 16 | - | 52 | - | - | 35 | - | - | 13 | - |
| 17 | 70 | - | - | 23 | - | - | - | - | 17 |

**Claims**

1. A pharmaceutical composition, comprising

   an active pharmaceutical ingredient (API),
   a carrier polymer, and
   tryptophan.

2. The pharmaceutical composition of claim 1, wherein the API and the carrier polymer are present at a w/w% ratio of about 1:99 to about 50:50, and tryptophan is present at a w/w% concentration of about 1% to about 30% of the total composition, preferably at an equimolar concentration with respect to the API.

3. The pharmaceutical composition according to claim 1 or 2, wherein the composition is capable of maintaining supersaturation of the API in a biorelevant medium.

4. The pharmaceutical composition according to any of claims 1 to 3, wherein the pharmaceutical composition is formulated as an amorphous solid dispersion (ASD).

5. The pharmaceutical composition according to any of claims 1 to 4, wherein the composition is capable of preventing recrystallization of the API in the amorphous solid dispersion (ASD).

6. The pharmaceutical composition according to any of claims 1 to 5, wherein the API is a Biopharmaceutics Classification System (BCS) Class II drug, a BCS Class IV drug, or any drug having low solubility.

7. The pharmaceutical composition according to any of claims 1 to 6, wherein the carrier polymer is a polyvinyl alcohol (PVA), preferably PVA 3-80, PVA 3-82, PVA 3-83, PVA 4-88, PVA 5-88, PVA 8-88, or PVA 5-74.

8. The pharmaceutical composition according to any of claims 1 to 7, wherein the pharmaceutical composition is obtained by hot-melt extrusion (HME) or spray drying (SD).

9. A method for producing a pharmaceutical composition, comprising the steps of

   a) mixing an API and a carrier polymer; and
   b) adding tryptophan.

10. The method according to claim 9, wherein the API and the carrier polymer are mixed in step a) at a w/w% ratio of about 1:99 to about 50:50, and tryptophan is added in step b) at a w/w% concentration of about 1% to about 30% of the total composition, preferably at an equimolar concentration with respect to the API.

11. The method according to any of claims 9 or 10, wherein the mixture obtained in step a) is an amorphous solid dispersion (ASD).

12. The method according to any one of claims 9 to 11, wherein the method is based on hot-melt extrusion (HME) or spray drying (SD).

13. The method according to any one of claims 9 to 12, wherein the API is a Biopharmaceutics Classification System (BCS) Class II drug, or a BCS Class IV drug, or any drug presenting low solubility.

14. The method according to any one of claims 9 to 13, wherein the carrier polymer is a polyvinyl alcohol (PVA), preferably PVA 3-80, PVA 3-82, 3-83, PVA 4-88, PVA 5-88, PVA 8-88, or PVA 5-74.

15. The method according to any one of claims 9 to 14, further comprising the steps of

    melting the mixture at about 210 °C under vacuum,
    cooling the mixture down to room temperature, and
    milling or cryomilling the resulting melt.

Fig. 1

Carvedilol - binary

Fig. 2

Carvedilol - ternary

Fig. 3

Carvedilol

Fig. 4

Ketoconazole

Fig. 5

Ketoconazole

Fig. 6

Ketoconazole

Fig. 7

**Ketoconazole**

Fig. 8

**Naproxen**

Fig. 9

**Naproxen**

Fig. 10

**Naproxen**

Fig. 11

**Naproxen**

Fig. 12

**Felodipin**

Fig. 13

Europäisches Patentamt

European Patent Office

Office européen des brevets

# EUROPEAN SEARCH REPORT

**Application Number**

EP 24 20 8829

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VEITH HEINER ET AL: "Combining crystalline and polymeric excipients in API solid dispersions - Opportunity or risk?", EUROPEAN JOURNAL OF PHARMACEUTICS AND BIOPHARMACEUTICS, ELSEVIER SCIENCE PUBLISHERS B.V., AMSTERDAM, NL, [Online] vol. 158, 6 December 2020 (2020-12-06), pages 323-335, XP086463993, ISSN: 0939-6411, DOI: 10.1016/J.EJPB.2020.11.025 Retrieved from the Internet: URL:https://www.sciencedirect.com/science/article/pii/S093964112030357X> [retrieved on 2020-12-06] * page 325 * * page 328 - page 329 * * the whole document * ----- | 1-15 | INV. A61K9/10 A61K47/18 A61K47/32 |
| Y | WO 2024/056773 A1 (MERCK PATENT GMBH [DE]) 21 March 2024 (2024-03-21) * page 1, line 5 - line 9 * * example 2 * * the whole document * ----- | 1-15 | |
| Y | US 2015/011525 A1 (BI YUNXIA [US] ET AL) 8 January 2015 (2015-01-08) * paragraph [0001] * * example 1 * * the whole document * ----- -/-- | 1-15 | |

TECHNICAL FIELDS SEARCHED (IPC)

A61K

The present search report has been drawn up for all claims

| Place of search | Date of completion of the search | Examiner |
|---|---|---|
| Munich | 10 March 2025 | Felder, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 20 8829

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | BYRN STEPHEN R ET AL: "Predictive and Accelerated Formulation Design Using Synchrotron Methods", AAPS PHARMSCITECH, SPRINGER INTERNATIONAL PUBLISHING, CHAM, [Online] vol. 20, no. 5, 29 April 2019 (2019-04-29) , pages 1-11, XP036822085, DOI: 10.1208/S12249-019-1375-2 Retrieved from the Internet: URL:https://link.springer.com/article/10.1 208/s12249-019-1375-2> [retrieved on 2019-04-29] * page 176 * * the whole document * ----- | 1-15 | |

**TECHNICAL FIELDS SEARCHED (IPC)**

The present search report has been drawn up for all claims

| Place of search | Date of completion of the search | Examiner |
|---|---|---|
| Munich | 10 March 2025 | Felder, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 20 8829

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2025

| Patent document cited in search report | Publication date | Patent family member(s) | | Publication date |
|---|---|---|---|---|
| WO 2024056773 A1 | 21-03-2024 | NONE | | |
| US 2015011525 A1 | 08-01-2015 | EP | 2755637 A1 | 23-07-2014 |
| | | US | 2015011525 A1 | 08-01-2015 |
| | | WO | 2013040187 A1 | 21-03-2013 |

EPO FORM P0459

For more details about this annex : see Official Journal of the European Patent Office, No. 12/82